# FASCICULE DE BREVET EUROPEEN

(11) **EP 1 303 259 B1**
(45) Date de publication et mention de la délivrance du brevet: **20.03.2013**
(21) Numéro de dépôt: 01938300.9
(22) Date de dépôt: 22.05.2001
(51) Int. Cl.: A61K 9/16

(54) **MICROSPHERES A LIBERATION PROLONGEE POUR ADMINISTRATION INJECTABLE ET PROCEDE DE PREPARATION**
MIKROSPHÄREN MIT VERLÄNGERTER FREISETZUNG ZUR INJIZIERBAREN VERABREICHUNG SOWIE HERSTELLUNGSVERFAHREN
PROLONGED RELEASE MICROSPHERES FOR INJECTION DELIVERY AND PREPARATION METHOD

(30) Priorité: 23.05.2000 FR 0006587
(43) Date de publication de la demande: 23.04.2003
(73) Titulaire: ETHYPHARM, 92213 St Cloud Cedex (FR)
(72) Inventeur: DULIEU, Claire, F-49000 Angers (FR); RICHARD, Joel, F-49160 Longue (FR); BENOIT, Jean-Pierre, F-49240 Avrille (FR)
(74) Mandataire: Regimbeau
(86) Numéro de dépôt international: PCT/FR2001/001575
(87) Numéro de publication internationale: WO 2001/089481

(56) Documents cités:
- EP-A- 0 257 368
- EP-A- 0 706 821
- WO-A-96/28143
- WO-A-98/15348
- WO-A1-00/04916
- WO-A1-00/10541
- WO-A1-01/12160
- WO-A1-01/45731
- WO-A1-98/31346
- WO-A2-01/51032
- WO-A2-01/85136
- P. G. DEBENEDETTI ET AL.: "application of supercritical fluids for the production of sustained delivery devices" JOURNAL OF CONTROLLED RELEASE, vol. 24, no. 1, 1 mai 1993 (1993-05-01), pages 27-44, XP000303918 Amsterdam (NL)

## Description

La présente invention concerne le domaine des microparticules destinées à être administrées par voie injectable, sous-cutanée ou intramusculaire.

Il existe à l'heure actuelle un besoin de formulations pharmaceutiques à libération prolongée, destinées à l'administration de protéines par voie injectable, qui soient dépourvues de toute trace de solvants organiques.

Des efforts intenses doivent être réalisés pour développer de nouveaux systèmes efficaces d'administration de protéines. Toutes les techniques classiques de préparation de systèmes microparticulaires injectables à libération contrôlée, que ce soit la préparation de microcapsules par la méthode d'émulsion (huile/eau)/évaporation de solvant (Hora et al., Pharm. Res., 7 (1990), 1190-1194 ; Jalil, R. et al., L. Microencapsulation, 7 (1990) 294-325), de coacervation en phase organique (Ruiz et al., Pharm. Res., 7 (1990) 928-934 ; Mc Gee, J.P. et al., J. Controlled Release, 34 (1995) 77-86) ou par la technique de double émulsion (eau/huile/eau)/évaporation de solvant (Ogawa, Y et al., Chem. Pharm. Bull., 36 (1988) 1095-1103) conduisent à l'utilisation de solvants organiques. Ceux-ci nécessitent des étapes de contrôle et de dosage précis des taux de solvants résiduels afin de limiter ces taux et d'éviter tout effet secondaire nuisible au patient. De plus, les autorités gouvernementales mettent en place des normes sévères pour éviter la contamination de l'environnement avec les solvants organiques inhérents aux procédés de production et limiter voire supprimer l'emploi de solvants organiques dans les compositions pharmaceutiques. Enfin, dans les formulations de protéine, des problèmes de dénaturation induits par le contact avec les solvants et des phénomènes indésirables d'adsorption aux interfaces solvants/eau peuvent apparaître.

La demande EP 257 368 a pour objet des microsphères pour administration parentérale contenant une protéine enrobée d'un corps gras ou d'une cire destinés à prolonger la libération de ladite protéine. Elles sont obtenues par nébulisation d'une formulation contenant de la protéine, un sel et un agent tensio-actif. La protéine et les additifs sont mélangés à la cire ou au corps gras en fusion avant de subir une nébulisation. Dans le cadre de ce procédé, la protéine est exposée à des températures élevées, de l'ordre de 75 à 80°C, nécessaires pour procéder à la fusion de la cire. L'application de telles températures provoque la dénaturation substantielle de la protéine. Les microsphères décrites dans la demande EP 257 368 sont destinées à un usage vétérinaire et les principes actifs protéiques formulés ne sont pas coûteux si bien que leur dénaturation substantielle au cours du procédé de préparation ne constitue pas un sérieux désavantage. L'enseignement de ce document n'est pas adapté à la formulation de principes actifs protéiques particulièrement sensibles à la chaleur et dont le prix de revient est tel que l'activité doit être préservée dans son intégralité.

La présente invention a pour objet des microsphères comme définies dans la revendication 1 destinées à être administrées par voie injectable comprenant un principe actif protéique et un agent enrobant le principe actif destiné à prolonger sa libération.

Les microparticules selon l'invention, contenant un principe actif protéique, se distinguent des microparticules de l'art antérieur par leur structure matricielle, par l'absence de toute trace de solvant organique et par le fait que le principe actif protéique n'est pas dénaturé.

Les microparticules selon l'invention sont dépourvues de toute trace de solvant organique, elles sont susceptibles d'être obtenues selon un procédé d'enrobage impliquant la mise en contact sous agitation du principe actif et de l'agent enrobant dans un fluide supercritique, ledit agent enrobant étant soluble dans le fluide supercritique. Le principe actif protéique est insoluble dans le fluide supercritique et il n'est pas dénaturé.

La taille moyenne des microparticules selon l'invention est comprise entre 0,1 et 150 µm.

Leur teneur en principe actif est comprise entre 0,5 et 50 % en poids, de préférence entre 3 et 20 % en poids.

Il a été mis en évidence que la mise en oeuvre d'un procédé de préparation de microparticules par une technique utilisant un fluide supercritique et un agent enrobant soluble dans ce fluide permet d'obtenir des microsphères aux propriétés avantageuses.

La demande EP 706 821 décrit l'enrobage de substances actives particulaires dans le cas où l'agent enrobant utilisé est soluble dans le CO₂ supercritique. Après solubilisation dans le CO₂ supercritique, l'agent enrobant est mis en contact avec la protéine à enrober dans un réacteur fermé sous agitation. Des modifications de pression et de température dans celui-ci conduisent à la désolvatation de l'agent enrobant et donc à sa précipitation sur la substance active. Cette méthode ne fait intervenir ni solvant organique, ni eau, et est réalisée à relativement basse température. Plus précisément, les particules de substance active sont mises en suspension dans le CO₂ supercritique, puis l'agent enrobant est dissous dans la suspension. La pression et/ou la température sont ensuite diminuées de façon contrôlée pour réduire la solubilité de l'enrobant dans le fluide supercritique et provoquer le dépôt de l'enrobant à la surface des particules de substance active. La couche d'enrobage peut être monomoléculaire ou atteindre jusqu'à 100 µpm d'épaisseur. La taille des particules encapsulées est comprise entre 20 nm et 500 µm. L'enrobant est un corps gras ou un polymère biodégradable soluble dans le CO₂ supercritique. Le dépôt de l'enrobant est effectué entre 30 et 45°C, entre 70 et 280 bars pendant 30 minutes à 4 heures, sous agitation.

La mise en oeuvre du procédé selon l'invention consiste à mettre un principe actif en suspension sous agitation dans un fluide supercritique contenant au moins un agent enrobant dissous dans celui-ci, puis à modifier les conditions de pression et/ou de température du milieu pour assurer la coacervation des particules, par désolvatation de l'agent enrobant autour des particules de principe actif, c'est-à-dire assurer la coacervation des particules par modification physico-chimique du milieu. Ce procédé conduit à des microparticules matricielles contenant plusieurs particules de principe actif protéique.

Il a été découvert dans le cadre de la présente invention qu'en adaptant la valeur de certains paramètres du procédé, notamment la teneur en principe actif équivalent au ratio principe actif/agent enrobant, le mode d'agitation, et le ratio agent enrobant/fluide supercritique, on obtient des microsphères aux propriétés avantageuses présentant une structure matricielle.

Le fluide supercritique préférentiellement utilisé est le CO₂ supercritique (CO₂SC), les conditions de fonctionnement initiales typiques de ce procédé sont d'environ 30 à 45°C et de 75 à 280 10⁵ Pa, bien que l'on puisse utiliser des valeurs plus élevées de l'un ou l'autre des deux paramètres ou les deux, à condition bien sûr que les valeurs plus élevées n'aient aucun effet nuisible ou de dégradation sur le principe actif en cours de revêtement, ni sur les agents enrobants.

Ce procédé implique la mise en suspension dans un autoclave, d'un principe actif non soluble dans le fluide supercritique, puis l'introduction dans cet autoclave de l'agent enrobant qui se trouve à l'état de soluté dans le fluide supercritique.

La pression et/ou la température sont ensuite modifiées de manière à diminuer la solubilité de l'agent enrobant dans le fluide. Ainsi l'affinité de l'agent enrobant pour le principe actif s'accroît de façon telle que cet enrobant s'adsorbe autour du principe actif. Une fois cet agent enrobant déposé sur le principe actif, l'autoclave est dépressurisé et les microparticules sont récupérées.

Pour mettre en oeuvre ce procédé, on place le principe actif à revêtir dans un autoclave équipé d'un agitateur, puis on pressurise le système en introduisant dans l'autoclave un fluide amené dans des conditions supercritiques. Finalement, on introduit le ou les agents enrobants dans l'autoclave, puis on modifie la température et/ou la pression à l'intérieur de l'autoclave d'une manière contrôlée et régulée de sorte à réduire progressivement la solubilité du ou des agents enrobants. Lorsque la solubilité de ce ou ces agents enrobants dans le fluide supercritique diminue, il(s) précipite(nt) et l'affinité de ces agents pour la surface du principe actif conduit à leur adsorption sur cette surface. Une variante de ce procédé consiste à placer l'agent enrobant dans l'autoclave avant d'y introduire le principe actif ou encore en y introduisant le principe actif puis un fluide susceptible de passer à l'état supercritique. La pressurisation de l'autoclave pour produire un état de fluide supercritique provoquera alors la dissolution de l'agent enrobant dans ledit fluide supercritique.

Les vitesses d'agitation peuvent varier entre 100 et 1000 tours/min, de préférence entre 150 et 450 t/min. Le choix de la vitesse d'agitation dépend de la taille du réacteur.

Une telle agitation assure la mise en suspension du principe actif dans le fluide supercritique lorsque celui-ci est introduit. Les conditions supercritiques sont assurées par une modification de la température et/ou de la pression à l'intérieur de l'autoclave. Ainsi, la température de l'autoclave est comprise entre 30 et 45°C et la pression est comprise entre 100 et 280 10⁵ Pa et de préférence entre 180 et 220 10⁵ Pa. L'agent enrobant est introduit dans l'autoclave en même temps que le fluide supercritique ou bien après l'introduction dans l'autoclave du fluide supercritique. En tous les cas pour assurer une bonne solubilisation de l'agent enrobant dans le fluide supercritique, on maintient le système à l'équilibre sous agitation, on établit la concentration adéquate en principe actif et en agent enrobant en fonction de la microparticule voulue et on laisse cet équilibre sous agitation pendant une heure environ. On module ensuite la température et la pression à une vitesse suffisamment lente pour transférer complètement le ou les agents enrobants du fluide supercritique à la surface du principe actif et on dépressurise le système pour isoler les microparticules que l'on retire de l'autoclave. Au moment de la dépressurisation, la vitesse d'agitation peut être conservée, diminuée ou arrêtée.

La concentration en agent enrobant dans le fluide supercritique est de préférence comprise entre 1,5 et 4,5 g/l, de préférence égale à 2 g/l.

Selon un mode de mise en oeuvre préféré de l'invention, un insert cylindrique est placé dans l'autoclave, et est vissé au couvercle avant fermeture. Le fluide subcritique est de préférence introduit par la partie haute de l'insert après fermeture de l'autoclave.

Cet insert est avantageusement équipé de deux frittages permettant l'entrée et la sortie du fluide supercritique. L'insert est de préférence pourvu d'un frittage annulaire dans sa partie haute, et d'un frittage discoïde constituant le fond dudit insert. Les deux frittages ont avantageusement une porosité inférieure à la taille des microsphères que l'on souhaite préparer.

L'insert permet de recueillir les microsphères contenant le principe actif. En fin de procédé il est dévissé et déplacé, éventuellement dans une enceinte contenant un gaz inerte lorsque le principe actif protéique est sensible à l'humidité, et est renversé afin de recueillir les microsphères. Il permet l'utilisation d'un gaz inerte propulseur pour faciliter la récupération des microsphères contenant le principe actif lorsque ledit principe actif est sensible à l'humidité.

L'agent enrobant entrant dans la composition des microsphères de l'invention, éventuellement choisi pour la mise en oeuvre de leur procédé de préparation
est un Gélucire^{®} (mélange de mono-, di- et triglycérides, et d'esters d'acide gras et de polyéthylène glycol).

Selon un mode de mise en oeuvre préféré, l'agent enrobant est un Gélucire^{®}, la température de l'autoclave est de l'ordre de 45°C, la pression de l'autoclave est de l'ordre de 200 bars et la vitesse d'agitation est de l'ordre de 450 t/min.

Le principe actif protéique peut être une protéine ou un peptide.

Les protéines entrant dans le cadre de la présente invention sont choisies parmi la protéine correspondant à l'hormone parathyroïde ("Parathyroïd hormone related protein"), l'hormone de croissance ("Growth hormone", GH), les interférons α, β ou γ, l'érythropoiétine α ou β (EPO), is facteur stimulateur de colonies de granulocytes ("Granulocyte colony stimulating factor", GCSF), le facteur stimulateur de colonies macrophages de granulocytes ("Granulocyte macrophage colony stimulating factor", GMCSF), le polypeptide PACAP ("Pituitary adenylate cyclase activating polypeptide"), le peptide vasoactif intestinal ("Vasoactive intestinal peptide", VIP), l'hormone libératrice de thyrotropine ("Thyrotropin releasing hormone", THR), l'hormone libératrice de thyrotropine ("Corticotropin releasing hormone", CRH), l'arginine vasopressine (AVP), l'angiotensine, l'insuline, la somatotropine, l'antigène HBS du virus de l'hépatite B, l'activateur de tissu plasminogène, les facteurs de coagulation VIII et IX, la glucosylcéramidase, la sargramostime, la lénograstine, la filgrastine, l'interleukine 2, la dornase α, la molgramostime, la PEG-L-asparaginase, la PEG-adenosine deaminase, l'hirudine, l'eptacog α (facteur VIIa de coagulation du sang humain) et les facteurs de croissances nerveuses (NGF, CNTF, BDNG, FGF, GDNF).

Une protéine particulièrement préférée dans le cadre de l'invention est l'érythropoiétine, une hormone protéique glycosylée qui a une action de facteur de croissance hématopoiétique. Elle est produite par génie génétique sous le nom d'époétine, et utilisée en clinique pour maintenir ou élever le taux de globules rouges du patient. Elle est indiquée dans les cas d'anémie, lors de l'hémodialyse d'insuffisants rénaux chroniques, en parallèle à une chimiothérapie, chez les malades HIV ou avant une intervention chirurgicale. Le traitement nécessite au moins trois injections par semaine.

Une autre protéine particulièrement préférée dans le cadre de l'invention est l'interféron alpha. Son spectre d'action est très large puiqu'il est utilisé à la fois pour ses propriétés antivirales, anticancéreuses et immunomodulatrices. Il est notamment utilisé pour le traitement de l'hépatite B, l'hépatite C, certaines leucémies et le syndrome de Kaposi. Le traitement comprend trois injections par semaine pendant 6 à 12 mois.

Des peptides, comme les dérivés de la LHRH ou de la somatostatine, la triptoréline, la bombésine, la calcitonine, l'hormone parathyroïde, le peptide libérateur de gastrine ("Gastrine releasing peptide", GRP), l'hormone libératrice d'hormone lutéinisante ("Luteinizing hormone releasing hormone", LHRH), le facteur libérateur d'hormone de croissance ("Growth hormone releasing factor", GRF), le dérivé de peptide Acétyl-Ser-Asp-Lys-Pro et l'amyline peuvent également être utilisés comme principe actif dans le cadre de la présente invention.

La taille des particules de principe actif protéique entrant dans la composition des microsphères est comprise entre 20 nm et 60 µm, de préférence entre 15 et 50 µm.

La présente invention a également pour objet un procédé de préparation des microsphères tel que décrit précédemment.

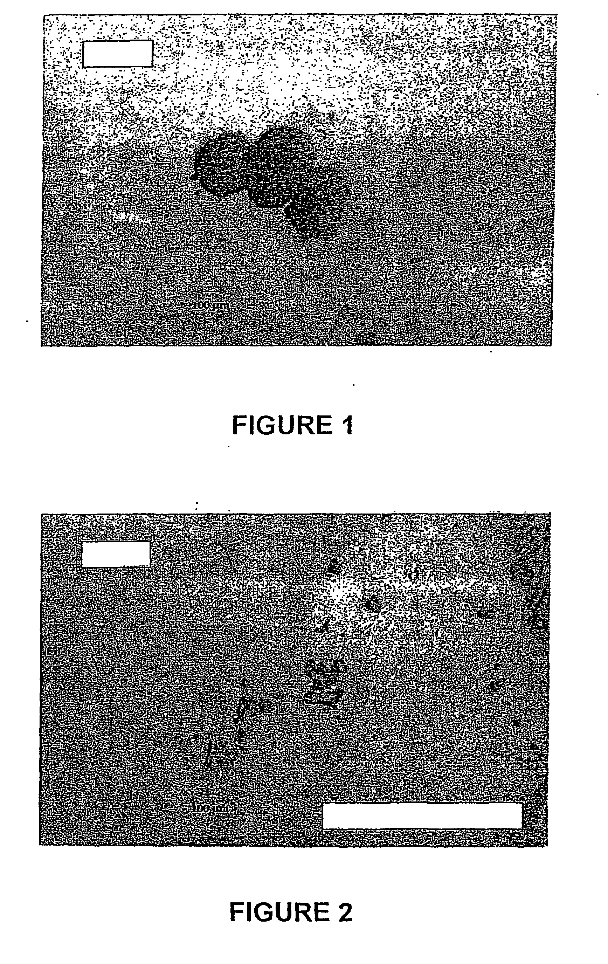

La figure 1 est une photographie au microscope optique de trois microsphères obtenues selon l'exemple 1.
La figure 2 permet de mettre en évidence la structure matricielle des microsphères de la figure 1. Après ajout de quelques gouttes de dichlorométhane (solvant de l'agent enrobant), une quinzaine de cristaux de BSA (insoluble dans le dichlorométhane) sont visibles.

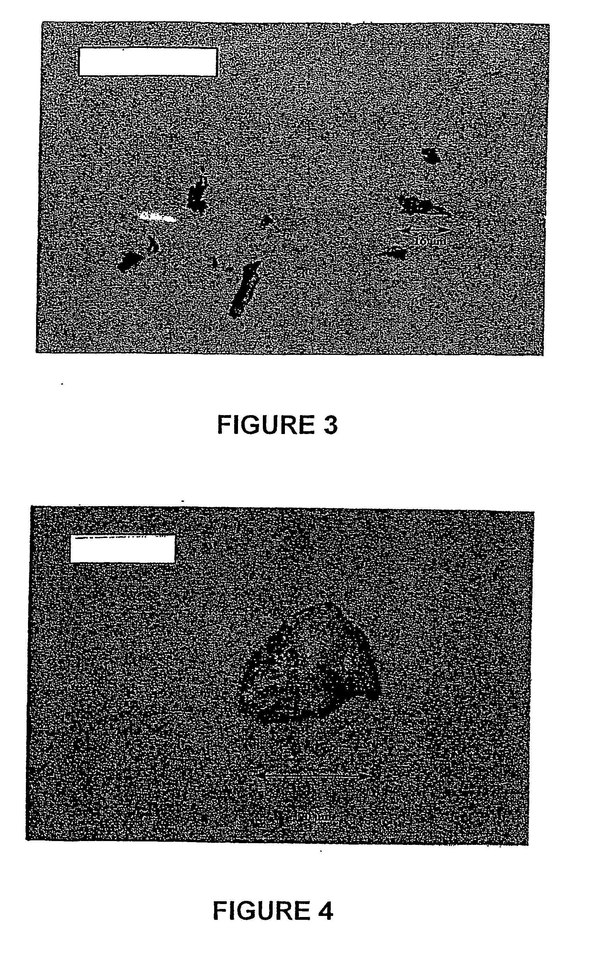

La figure 3 est une photographie au microscope optique de particules d'érythropoïétine utilisés dans l'exemple 2, avant leur enrobage.
La figure 4 est une photographie au microscope optique d'une microsphère contenant des particules d'érythropoïétine obtenue selon l'exemple 2.

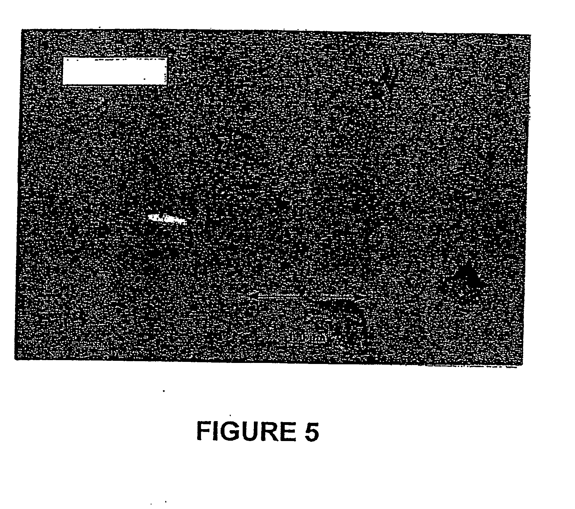

La figure 5 permet de mettre en évidence la structure matricielle de la microsphère de la figure 4. Après l'ajout de quelques gouttes de dichlorométhane (solvant de l'agent enrobant), trois cristaux d'érythropoïétine sont visibles.

### Exemple 1 : Microsphères de Gélucire^{®} 50/02 contenant la protéine modèle albumine de sérum bovin (BSA)

### Matériel

Autoclave de 300 mL muni d'un insert de volume 180 mL, poreux (10 µm) en haut et en bas. Autoclave muni d'une double enveloppe pour la régulation de température : circulation d'huile de silicone, chauffée ou refroidie par un bain thermostaté. Agitation dans l'autoclave : moteur régulé par contrôle PID, axe avec un mobile à deux pales en forme d'ancre marine.

### Produits

- BSA Fraction V (Sigma A-7906) broyée au mortier et tamisée. Fraction 32 à 50 µm : 118,45 mg.
- Gélucire^{®} 50/02 (Gattefossé) masse cireuse réduite en copeaux à la spatule : 452,8 mg.

### Procédure

Les deux produits sont placés au fond de l'insert. L'autoclave est fermé et mis sous agitation à 495 t/min. Les séquences successives de formation des microsphères sont alors les suivantes :
- injection de CO₂ par équilibration avec le réservoir puis la colonne jusqu'aux conditions initiales de 24,9°C (T) et 99 bars.
- chauffage de l'autoclave par circulation d'eau chaude dans la double enveloppe. Le chauffage est régulé par PID; Durée du chauffage 34 min.
- maintien à l'équilibre des paramètres température/pression à 45,1°C et 183 bars. Ces conditions sont maintenues pendant 66 min, agitation 495 t/min.
- refroidissement par circulation de fluide dans la double enveloppe.
   Durée 41 min, jusqu'à : T = 20°C, P = 79 bars.
- décompression dans un récipient-tampon jusqu'à : T = 25°C, P atmosphérique. Durée = 7 min.

Les particules enrobées sont recueillies dans l'insert. On en recueille environ 150 mg.

### Exemple 2 : Microsphères de Gélucire^{®}50/02 contenant une préparation commerciale d'érythropoïétine solide (Hémax^{®})

### Matériel

Identique à celui utilisé dans l'exemple 1.

### Produits

- Hémax^{®} 2000 UI : 64 mg.
- Gélucire^{®} 50/02 (Gattefossé) masse cireuse réduite en copeaux à la spatule : 259 mg.

### Procédure

Les deux produits sont placés au fond de l'insert. L'autoclave est fermé et mis sous agitation à 460 t/min. Les séquences successives de formation des microsphères sont alors les suivantes :
- injection de CO₂ par équilibration avec le réservoir puis la colonne jusqu'aux conditions initiales de T = 23°C, P = 100 bars.
- chauffage de l'autoclave par circulation d'eau chaude dans la double enveloppe. Le chauffage est régulé par un système PID. Durée du chauffage 24 min.
- stabilisation des température/pression à 45°C et 203 bars. Ces conditions sont maintenues pendant 65 min, agitation 460 t/min.
- refroidissement par circulation de fluide dans la double enveloppe.
   Durée 49 min, jusqu'à : T = 17°C, P = 74 bars.
- décompression par ouverture à l'évent jusqu'à : T = 25°C, P atmosphérique. Durée = 25 min.
- récupération des particules sous atmosphère d'azote. On en récupère 150 à 160 mg.

### Exemple 3 (non conforme à l'invention) : Microsphères de Witepsol^{®} E85 contenant une préparation

### commerciale d'érythropoétine solide (Hémax^{®}).

### Matériel

Identique à celui utilisé dans l'exemple 1.

Produits (quantités)
- Hémax^{®} 2000 UI : 150,2 mg
- Witepsol^{®} E85 (Condéa) glycéride d'acide gras C₁₀-C₁₈, masse cireuse réduite en copeaux à la spatule : 450,2 mg.

### Procédure

Les deux produits sont placés au fond de l'insert. L'autoclave est fermé et mis sous agitation à 460 t/min. Les séquences successives de formation des microsphères sont alors les suivantes :
- injection de CO₂ par équilibration avec le réservoir puis avec la colonne jusqu'aux conditions initiales de T=22°C, P=131 bars.
- chauffage de l'autoclave par circulation d'eau chaude dans la double enveloppe. Le chauffage est régulé par un système PID. Durée du chauffage 46 minutes.
- stabilisation des température/pression à 35°C et 199 bars. Ces conditions sont maintenues pendant 60 minutes, agitation 460 t/min.
- refroidissement par circulation d'eau froide dans la double enveloppe. Durée 40 min, jusqu'à : T=4,9°C, P=64 bars.
- décompression par ouverture à l'évent jusqu'à 25°C et P atomosphérique. Durée 21 min.
- récupération des particules sous atmosphère d'azote.

On récupère 306,2 mg d'une poudre fluide de microparticules contenant la protéine.

### Exemple 4 (non conforme à l'invention): Microsphères de Suppocire^{®} ND contenant une préparation

### commerciale d'érythropoétine solide (Hémax^{®}).

### Matériel

Identique à celui utilisé dans l'exemple 1.

Produits (quantités)
- Hémax^{®} 2000 UI : 173,3 mg
- Suppocire^{®} ND (Gattefossé) mélange de mono-, di- et triglycérides, masse cireuse réduite en copeaux à la spatule : 693,76 mg.

### Procédure

Les deux produits sont placés au fond de l'insert. L'autoclave est fermé et mis sous agitation à 460 t/min. Les séquences successives de formation des microsphères sont alors les suivantes :
- injection de CO₂ par équilibration avec le réservoir puis avec la colonne jusqu'aux conditions initiales de T=22°C, P=129 bars.
- chauffage de l'autoclave par circulation d'eau chaude dans la double enveloppe. Le chauffage est régulé par un système PID. Durée du chauffage 26 min.
- stabilisation des température/pression à 35°C et 200 bars. Ces conditions sont maintenues pendant 60 minutes, agitation 460 t/min.
- refroidissement par circulation d'eau froide dans la double enveloppe. Durée 45 min, jusqu'à : T=22°C, P=61 bars.
- décompression par ouverture à l'évent jusqu'à P atmosphérique.
   Durée = 31 min.
- récupération des particules sous atmosphères d'azote.

On récupère 439,76 mg d'une poudre fluide de microparticules contenant la protéine.

### Exemple 5 (non conforme à l'invention) : Microsphères de Dynasan contenant une protéine modèle

### albumine de sérum bovin (BSA).

### Matériel

Autoclave de 1 L muni d'une double enveloppe pour la régulation de la température : circulation d'eau chaude ou froide par un bain thermostaté. Agitation dans l'autoclave, axe avec un mobile en forme d'ancre marine.

### Produits

- BSA Fraction V (Sigma A - 7906) broyée au mortier et tamisée. Fraction 50-125 µm : 400,30 mg.
- Dynasan^{®} 114 (Condéa), triglycéride d'acide myristique : 840,49 mg.
- Dynasan^{®} 116 (Condéa), triglycéride d'acide palmitique : 700,6 mg.
- Dynasan^{®} 118 (Condéa), triglycéride d'acide stéarique : 59,85 mg.

### Procédure

Les quatre produits sont placés au fond de l'autoclave. L'autoclave est fermé et mis sous agitation à 210 t/min. Les séquences successives de formation des microsphères sont alors les suivantes :
- injection de CO₂ par équilibration avec le réservoir puis avec la colonne jusqu'aux conditions initiales de T=25°C, P= 134 bars.
- chauffage de l'autoclave par circulation d'eau chaude dans la double enveloppe. Le chauffage est régulé par un système PID. Durée du chauffage : 23 minutes.
- stabilisation des température/pression à 45°C et 250 bars. Ces conditions sont maintenues pendant 60 minutes, agitation 210 t/min.
- refroidissement par circulation d'eau froide dans la double enveloppe. Durée 37 min, jusqu'à : T=15°C, P= 72 bars.
- arrêt de l'agitation,
- décompression par ouverture à l'évent jusqu'à P atmosphérique.
   Durée =30 min.
- récupération des particules sous atmosphère d'azote.

On récupère 1,34 g d'une poudre fluide de microparticules contenant la protéine.

### Exemple 6 : Microsphères de Gélucire^{®} 50/02 contenant une protéine

### modèle albumine de sérum bovin (BSA).

### Matériel

Autoclave de 60 l muni d'un insert de volume 50 L. Autoclave muni d'une double enveloppe pour la régulation de la température : circulation d'eau chaude ou froide par un bain thermostaté. Agitation dans l'autoclave, axe avec trois mobiles en forme d'hélice à 4 pâles.

Produits (quantités)
- BSA Fraction V (Sigma A - 7906) broyée au mortier et tamisée. Fraction 50-125 µm : 80 g.
- Gélucire^{®} 50/02 (Gattefossé), masse cireuse réduite en copeaux à la spatule : 180 g.

### Procédure

Les deux produits sont placés au fond de l'insert. L'autoclave est fermé et mis sous agitation à 150 t/min. Les séquences successives de formation des microsphères sont alors les suivantes :
- injection de CO₂ par équilibration avec le réservoir puis avec la colonne jusqu'aux conditions initiales de T=23°C, P= 90 bars.
- chauffage de l'autoclave par circulation d'eau chaude dans la double enveloppe.
- stabilisation des température/pression à 45°C et 200 bars. Ces conditions sont maintenues pendant 60 minutes, agitation 150 t/min.
- refroidissement par circulation d'eau froide dans la double enveloppe. Durée 50 min, jusqu'à : T=18°C, P= 63 bars.
- arrêt de l'agitation,
- décompression par ouverture à l'évent jusqu'à P atmosphérique.
   Durée = 6h.
- récupération des particules sous atmosphère d'azote.

On récupère 120 g d'une poudre fluide de microparticules contenant la protéine.

## Revendications

1. Microsphères de structure matricielle, dépourvues de toute trace de solvant organique, consistant en un principe actif protéique non dénaturé et un agent enrobant destiné à prolonger sa libération, et obtenues par coacervation de l'agent enrobant en présence d'un fluide supercritique dans un autoclave, pour leur utilisation comme médicament destiné à être injecté par voie sous cutanée ou intramusculaire,
ledit agent enrobant étant un mélange de mono-, di-, et triglycérides, et d'esters d'acide gras et de polyéthylène glycol :
- ladite coacervation comprenant les étapes de mise en suspension et dissolution sous agitation respectivement du principe actif et de l'agent enrobant dans le fluide supercritique, et de
- modification de la température et/ou de la pression pour désolvater de façon contrôlée l'agent enrobant et provoquer sa coacervation sur le principe actif, l'agitation étant maintenue.

2. Microsphères pour leur utilisation comme médicament selon la revendication 1, **caractérisées en ce que** leur taille moyenne est comprise entre 0,1 et 150 µm.

3. Microsphères pour leur utilisation comme médicament selon l'une des précédentes revendications, **caractérisées en ce que** leur teneur en principe actif est comprise entre 0,5 et 50 % en poids, de préférence entre 3 et 20 % en poids.

4. Microsphères pour leur utilisation comme médicament selon l'une des revendications précédentes, **caractérisées en ce que** le principe actif protéique est une protéine choisie parmi la protéine correspondant à l'hormone parathyroïde, l'hormone de croissance, le facteur stimulateur de colonies de granulocytes, le facteur stimulateur de colonies macrophages de granulocytes, le peptide vasoactif intestinal, l'hormone libératrice de thyrotropine, l'arginine vasopressine, l'angiotensine, l'insuline, la somatotropine, l'antigène HBS du virus de l'hépatite B, l'activateur de tissu plasminogène, les facteurs de coagulation VIII et IX, la glucosylcéramidase, la sargramostime, la lénograstine, la filgrastine, l'interleukine 2, la dornase α, la molgramostime, la PEG-L-asparaginase, la PEG-adenosine deaminase, l'hirudine, l'eptacog α, l'erythropoietine et les facteurs de croissances nerveuses.

5. Microsphères pour leur utilisation comme médicament selon la revendication 4, **caractérisées en ce que** le principe actif est l'érythropoiétine.

6. Microsphères pour leur utilisation comme médicament selon l'une des revendications 1 à 3, **caractérisées en ce que** le principe actif protéique est un peptide choisi parmi la triptoréline, la bombésine, la calcitonine, l'hormone parathyroïde, le peptide libérateur de gastrine, l'hormone libératrice d'hormone lutéinisante, le facteur libérateur d'hormone de croissance, et l'amyline.

7. Microsphères pour leur utilisation comme médicament selon l'une des revendications précédentes, **caractérisées en ce que** la concentration en agent enrobant dans le fluide supercritique est comprise entre 1,5 et 4,5 g/l, de préférence égale à 2 g/l environ.

8. Microsphères pour leur utilisation comme médicament selon l'une des revendications précédentes, **caractérisées en ce qu'**elles sont obtenues à une température de coacervation comprise entre 30 et 45°C, à une pression de coacervation comprise entre 100 et 280 10⁵ Pa, de préférence entre 180 et 220 10⁵ Pa, et sous une agitation dont la vitesse est comprise entre 100 et 1000 t/min, de préférence égale à 450 t/min.

9. Microsphères pour leur utilisation comme médicament selon l'une des revendications précédentes, **caractérisées en ce qu'**un insert est placé dans l'autoclave , et que la mise en suspension et la dissolution du principe actif et de l'agent enrobant sont respectivement réalisées dans l'insert.

10. Microsphères pour leur utilisation comme médicament selon la revendication 9, **caractérisées en ce que** ledit insert est muni de deux frittages permettant l'entrée et la sortie du fluide supercritique.

## Claims

1. Microspheres having a matrix structure, free of any trace of organic solvent, consisting of a non-denatured protein active ingredient and a coating agent intended to prolong its release, and obtained by coacervation of the coating agent in the presence of a supercritical fluid in an autoclave, for use as medicament intended to be injected by the subcutaneous or intramuscular route,
the said coating agent being a mixture of mono-, di- and triglycerides, and of fatty acid esters and of polyethylene glycol:
- the said coacervation comprising the steps consisting of the suspension and dissolution, with stirring, respectively of the active ingredient and of the coating agent in the supercritical fluid, and of
- the modification of the temperature and/or of the pressure in order to desolvate the coating agent in a controlled manner and to cause its coacervation on the active ingredient, the stirring being maintained.

2. Microspheres for use as medicament according to Claim 1, **characterized in that** their mean size is between 0.1 and 150 µm.

3. Microspheres for use as medicament according to one of the preceding claims, **characterized in that** their content of active ingredient is comprised between 0.5 and 50% by weight, preferably between 3 and 20% by weight.

4. Microspheres for use as medicament according to one of the preceding claims, **characterized in that** the protein active ingredient is a protein chosen from the parathyroid hormone, the growth hormone, the granulocyte colony stimulating factor, the granulocyte macrophage colony stimulating factor, the vasoactive intestinal peptide, the thyrotropin releasing hormone, the arginine vasopressin, the angiotensin, the insulin, the somatotropin, the HBS antigen of the hepatitis B virus, the plasminogen tissue activator, the coagulation factors VIII and IX, the glucosylceramidase, the sargramostim, the lenograstin, the filgrastin, the interleukin-2, the dornase-α, the molgramostim, the PEG-L-asparaginase, the PEG-adenosin deaminase, the hirudin, the eptacog-α, the erythropoietin and the nerve growth factors.

5. Microspheres for use as medicament according to Claim 4, **characterized in that** the active ingredient is erythropoietin.

6. Microspheres for use as medicament according to one of Claims 1 to 3, **characterized in that** the protein active ingredient is a peptide chosen from triptorelin, bombesin, calcitonin, parathyroid hormone, gastrin releasing peptide, luteinizing hormone releasing hormone, growth hormone releasing factor and amylin.

7. Microspheres for use as medicament according to one of the preceding claims, **characterized in that** the concentration of coating agent in the supercritical fluid is comprised between 1.5 and 4.5 g/l, preferably equal to about 2 g/l.

8. Microspheres for use as medicament according to one of the preceding claims, **characterized in that** they are obtained at a coacervation temperature comprised between 30 and 45°C, at a coacervation pressure comprised between 100 and 280 × 10⁵ Pa, preferably between 180 and 220 × 10⁵ Pa, and with stirring, the speed of which is comprised between 100 and 1000 rpm, preferably equal to 450 rpm.

9. Microspheres for use as medicament according to one of the preceding claims, **characterized in that** an insert is placed in the autoclave and that the suspension and dissolution of the active ingredient and of the coating agent are respectively carried out in the insert.

10. Microspheres for use as medicament according to Claim 9, **characterized in that** the said insert is provided with two sinters allowing the inflow and outflow of the supercritical fluid.

## Patentansprüche

1. Mikrosphären mit Matrizenstruktur, frei von jeder Spur an organischem Lösemittel, bestehend aus einem nicht denaturierten Proteinwirkstoff und einem Umhüllungsmittel das seine Freisetzung verlängern kann, und erhalten durch Koacervierung des Umhüllungsmittels in Gegenwart eines super-kritischen Fluids in einem Autoklaven, zur Verwendung als Arzneimittel das über den subkutanen oder intramuskulären Weg injiziert werden kann, wobei das Umhüllungsmittel eine Mischung aus Mono-, Di- und Triglyzerin und Fettsäureestern und Polyethylenglycol ist:
- wobei die Koacervierung die Schritte des Suspendierens und Auflösens unter Schütteln des Wirkstoffs beziehungsweise des Umhüllungsmittels in dem superkritischen Fluid umfasst, und
- Modifizieren der Temperatur und / oder des Drucks um auf kontrollierte Art und Weise das Umhüllungsmittel aufzulösen und seine Koacervierung auf dem Wirkstoff zu provozieren, wobei das Schütteln aufrecht erhalten wird.

2. Mikrosphären zur Verwendung als Arzneimittel nach Anspruch 1, **dadurch gekennzeichnet, dass** der mittlere Umfang zwischen 0,1 und 150 µm liegt.

3. Mikrosphären zur Verwendung als Arzneimittel nach einem beliebigen der vorherigen Ansprüche, **dadurch gekennzeichnet, dass** ihr Gehalt an Wirkstoff zwischen 0,5 und 50 Gewichts %, vorzugsweise zwischen 3 und 20 Gewichts % liegt.

4. Mikrosphären zur Verwendung als Arzneimittel nach einem beliebigen der vorherigen Ansprüche, **dadurch gekennzeichnet, dass** der Proteinwirkstoff ein Protein ist ausgewählt aus dem Protein entsprechend dem Parathormon, dem Wachstumshormon, dem Granulozytenkolonien stimulierenden Faktor, dem Granulozyten Makrophagenkolonien stimulierenden Faktor, dem vasoaktiven intestinalen Peptid, dem Freisetzungshormon des Thyrotropins, dem Arginin Vasopressin, dem Angiotensin, dem Insulin, dem Somatotropin, dem HBS Antigen des Hepatitis B Virus, dem gewebespezifischen Plasminogen-Aktivator, den Koagulationsfaktoren VIII und IX, der Glucosylceramidase, dem Sargramostim, dem Lenograstim, dem Filgrastim, dem Interleukin 2, der Dornase a, dem Molgramostim, der PEG-L-Asparaginase, der PEG-Adenosin Deaminase, dem Hirudin, dem Eptacog a, dem Erythropoietin und den Nervenwachstumsfaktoren.

5. Mikrosphären zur Verwendung als Arzneimittel nach Anspruch 4, **dadurch gekennzeichnet, dass** ihr Wirkstoff Erythropoietin ist.

6. Mikrosphären zur Verwendung als Arzneimittel nach einem der Ansprüche 1-3, **dadurch gekennzeichnet dass** ihr Proteinwirkstoff ein Peptid ist ausgewählt aus dem Triptorelin, dem Bombesin, dem Calcitonin, dem Parathormon, dem Freisetzungspeptid Gastrin, dem Freisetzungshormon des luteinisierenden Hormons, dem Freisetzungsfaktor des Wachstumshormons und dem Amylin.

7. Mikrosphären zur Verwendung als Arzneimittel nach einem beliebigen der vorherigen Ansprüche, **dadurch gekennzeichnet, dass** die Konzentration an Umhüllungsmittel in dem superkritischen Fluid zwischen 1,5 und 4,5 g/l liegt, vorzugsweise etwa 2 g/l ist.

8. Mikrosphären zur Verwendung als Arzneimittel nach einem beliebigen der vorherigen Ansprüche, **dadurch gekennzeichnet, dass** sie bei einer Koacevierungstemperatur zwischen 30 und 45°C, bei einem Koacevierungsdruck der zwischen 100 und 280 10⁵ Pa liegt, vorzugsweise zwischen 180 und 220 10⁵ Pa und unter Schütteln bei einer Geschwindigkeit die zwischen 100 und 1000 t/min liegt, vorzugsweise 450 t/min ist, erhalten werden.

9. Mikrosphären zur Verwendung als Arzneimittel nach einem beliebigen der vorherigen Ansprüche, **dadurch gekennzeichnet, dass** ein Einsatzteil in den Autoklav platziert wird und das Suspendieren und das Auflösen des Wirkstoffs beziehungsweise des Umhüllungsmittels in dem Einsatzteil durchgeführt werden.

10. Mikrosphären zur Verwendung als Arzneimittel nach Anspruch 9, **dadurch gekennzeichnet, dass** das Einsatzteil mit zwei Sinterungen ausgestattet ist, die Zu- und Ablauf des superkritischen Fluids erlauben.
